(19) Europäisches Patentamt
European Patent Office
Office européen des brevets

(11) **EP 4 548 970 A2**

(12) **EUROPEAN PATENT APPLICATION**

(43) Date of publication:
07.05.2025 Bulletin 2025/19

(21) Application number: 25163216.2

(22) Date of filing: **15.12.2022**

(51) International Patent Classification (IPC):
**A61P 1/04** (2006.01)

(52) Cooperative Patent Classification (CPC):
(C-Sets available)
**A61K 9/0058; A61K 31/734; A61K 33/00;
A61K 33/10; A61P 1/04;** A61K 9/06; A61K 9/2826
(Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Validation States:
**MA**

(30) Priority: **22.12.2021 EP 21383196**

(62) Document number(s) of the earlier application(s) in
accordance with Art. 76 EPC:
**22839643.8 / 4 452 241**

(27) Previously filed application:
**15.12.2022 EP PCT/EP2022/086016**

(71) Applicant: **Chemo Research SL
28050 Madrid (ES)**

(72) Inventors:
• **RIZO, José Miguel
28043 Madrid (ES)**
• **VICEDO, Laura
03680 Alicante (ES)**

(74) Representative: **Ponti & Partners, S.L.P
Edifici PRISMA
Av. Diagonal núm. 611-613, Planta 2
08028 Barcelona (ES)**

Remarks:
This application was filed on 12-03-2025 as a
divisional application to the application mentioned
under INID code 62.

(54) **A NON-SWALLOWED, ANTACID CHEWING GUM PRODUCT, A PROCESS FOR ITS PREPARATION AND USES THEREOF**

(57) The present invention provides a non-swallowed, antacid chewing gum tablet comprising various antacid components with improved organoleptic properties.

The invention also provides an extrusion process for obtaining the non-swallowed, antacid chewing gum tablet as well as the use of the extruded chewing gum tablet as an antacid medicament, and for the prevention and/or the treatment of antacid diseases.

**EP 4 548 970 A2**

(52) Cooperative Patent Classification (CPC): (Cont.)

C-Sets
**A61K 31/734, A61K 2300/00;**
**A61K 33/00, A61K 2300/00;**
**A61K 33/10, A61K 2300/00**

**Description**

**Field of the invention**

The present invention relates to non-swallowed, antacid chewing gums. More particularly, the invention relates to a novel chewing gum product, which is capable of producing a potent antacid effect without the need to swallow it while maintaining adequate organoleptic and antacid release properties. The invention also relates to a process for preparing the chewing gum product of the invention as well as its uses.

**Background of the invention**

Several chewing gums have been described in the art as having antacid effect. However, the large amount of antacid used for effectiveness reason does not lend itself to give a good tasting product chewing gum. Moreover, the high amounts of sugar in some products is not consumer acceptable.

WO 02/17966, WO 02/17731 or WO02/051259 of Wrigley disclose coated chewing gums formulations having a coating with antacid properties.

Particularly, WO 02/17966 and WO 02/17731 use calcium carbonate as antacid component in the coating, and WO02/051259 uses an antacid component other than calcium carbonate in the coating.

WO 02/17966 discloses a method of making antacid coated chewing gum products. In accordance with WO 02/17966, it is believed that providing the antacid in a chewing gum coating that produces an increased viscosity in the saliva when the coating is dispersed and dissolved upon chewing makes the antacid more effective. However, when the coating contains high levels of calcium carbonate (about 25% to 60%), the polyols generally lack sufficient sweetness to give a good tasting product. Direct viscosity measurements of saliva is difficult due to the variable nature of saliva. The antacid coating comprises a bulk sweetener; calcium carbonate and a binding agent. The use of a binding agent increases the viscosity of the coating solution and, when chewed, increases the residence time of the antacid in the gastrointestinal tract. Therefore, it discloses a coating syrup that contains suspended calcium carbonate in combination with a high intensity sweetener and a binding agent.

Although WO 02/17966 mentions twice alginate, the alginate is mentioned as a binding agent in the coating for the purposes of the binding agent and in combination with calcium carbonate and a bulk sweetener. No antacid sodium alginate in the core is described or suggested, furthermore, no percentages are mentioned.

Thus, WO 02/17966 discloses that the calcium carbonate is combined with a bulk sweetener and a binding agent to make a more viscous solution than previously used coating ingredients. This combination results in an increase in the residence time of the antacid in the gastrointestinal tract, therefore, giving extended relief.

In a similar way, Wrigley's WO 01/95738, also discloses a method for making coated chewing gum products, but in this document, it is more specifically described how is making the coating. The method of making coated chewing gum products comprises providing a first coating syrup comprising a bulk sweetener; providing a second coating syrup separate from the first coating syrup and comprising a high-intensity sweetener. When an antacid is included in the coating, it is included in the first coating syrup which is essentially free of high-intensity sweeteners, meaning that the syrup does not contain any, or contains such a low amount of high-intensity sweetener that the high-intensity sweetener does not provide a perceptible additional sweetness. Although WO 01/95738 mentions twice alginate, the alginate is as a binding agent in the coating in combination with calcium carbonate, a bulk sweetener and a high-intensity sweetener. No antacid sodium alginate in the core is described or suggested, furthermore, no percentages are mentioned.

In WO 02/17731, calcium carbonate is used having an average particle size of greater than about 3 microns to make an antacid coating having a prolonged effectiveness.

WO02/051259 uses antacid compounds other than calcium carbonate as stated above. The antacid compound other than calcium carbonate is selected from the group consisting of aluminum salts, bismuth salts, magnesium salts, sodium bicarbonate, potassium bicarbonate, potassium citrate, sodium potassium tartrate, tricalcium phosphate and mixtures thereof. The antacid compound/composition is suspended in a coating syrup, then applied to the cores for obtaining a coated chewing gum with antacid properties.

Other solid preparations different from chewing gums have been also disclosed in the art containing sodium alginate as antacid component.

Solid preparations such as tablets, sold under the trade mark GAVISCON of Reckitt, are formulated for oral administration. On ingestion, the medicinal product reacts rapidly with gastric acid to form a raft of alginic acid gel having a near neutral pH and studies have shown that the raft interacts with and caps the acid pocket in the stomach, reducing oesophageal acid exposure. The raft floats on the stomach contents effectively impeding gastro-oesophageal reflux, for up to 4 hours, and protecting the oesophagus from acid, pepsin and bile. In severe cases the raft itself may be refluxed into the oesophagus, in preference to the stomach contents, and exert a demulcent effect. Calcium carbonate neutralises gastric acid to provide fast relief from indigestion and heartburn. This effect is increased by the addition of sodium bicarbonate which also has a neutralising action. The total neutralising capacity of the product at the lowest dose of two tablets is approximately 10mEqH+. However, these chewable tablets need to be swallowed after being chewed.

WO03/068246 of Reckitt discloses also solid, ingestible compositions. In this case, the solid, ingestible composition comprises: alginate; sodium bicarbonate and/or calcium carbonate and a $C_2$-$C_5$ polyol or poly($C_2$-$C_5$ alkylene glycol) having a molecular weight of at least 6,000. Alginate is used for non-foaming purposes in view of alginic acid. However, alginate is sticky and causes the composition to stick to the palate and especially to the teeth being the presence of $C_2$-$C_5$ polyol or poly($C_2$-$C_5$ alkylene glycol) essential to mitigate the drawbacks of alginate. Alginate is administered in an amount of from 100 to 2,000 mg per dose, and in the composition the polyol or poly-alkylene glycol and the alginate are in a weight ratio of from 2:1 to 1:25. Method of preparation includes: ingredients together in particulate form are mixed and then granulate or agglomerate. Alternatively, one or more of the ingredients can be added after granulation. Comparative example 1 and examples 3 to 7 reveal that the best results were obtained using the highest amount of PEG. However, an accurate control of the amounts of $C_2$-$C_5$ polyol or poly($C_2$-$C_5$ alkylene glycol) should be performed since the amount of this component which is ingested is limited by regulatory authorities.

Moreover, when alginate is used, the amount is limited for organoleptic reasons as disclosed in WO 2000/030466 to Wrigley. This patent application discloses chewing gums including up to 1% of alginates to increase flavor release and calcium carbonate as filler component.

In view of the above-mentioned drawbacks in the art, there is still the need to solve the problem of formulating alginate in a chewing gum.

Sodium alginate has been used to prevent reflux by forming a raft in the stomach that acts as a physical barrier against the movement of the stomach contents. However, these formulations have not been totally consumer acceptable; the large amount of this antacid does not lend itself to giving a good tasting product. Moreover, the use of concentrated alginate in the oropharyngeal region may cause a "gag reflex".

The amount of sodium alginate is limited in the chewing gums formulations of the art.

Thus, there is a need in the art to provide an antacid product totally consumer acceptable, which gives a good tasting product, with improved antacid effect, that does not need to be swallowed and that can be obtained at industrial scale.

An object of the present invention is, therefore, to provide a chewing gum product having improved antacid action and improved organoleptic properties compared to the chewing gum tablets in the art, and in which the gag reflex deriving from using alginate in the formulation is minimized or removed. The chewing gum product of the invention is formulated to be chewed, and not to be swallowed thus avoiding the discomfort that swallowing provokes in the consumer suffering acid regurgitation, heartburn, dyspepsia, gastric reflux, oesophagitis and/or peptic ulceration.

There is also an object of the present invention to provide a process for obtaining the chewing gum product of the invention that can be scaled at industrial scale.

## Summary of the invention

The present invention was made in view of the prior art described above, and the first object of the present invention is to provide an antacid chewing gum product which is formulated and obtained to be chewed, and not to be swallowed. This is a main goal of the invention which provides various advantages over prior art as well as being especially suitable for a group of patients having difficulty swallowing.

- Antacid chewing gum -

Accordingly, the present invention provides a chewing gum tablet, for antacid purposes that is non-ingestible, comprising a core and a coating. The non-swallowed, antacid chewing gum tablet comprises a core and a coating, and is characterized in that the tablet comprises calcium carbonate as antacid active ingredient, and the core comprises:

- gum base,
- sodium alginate as antacid active ingredient, and
- sodium bicarbonate as antacid active ingredient,

wherein the sodium alginate is from 12.0 to 25.0% w/w of the core.

Preferable non-swallowed, antacid chewing gum tablet comprises a core and a coating, and is characterized in that the tablet comprises calcium carbonate as antacid active ingredient, and the core comprises:

- gum base in a percentage between 32.0% and 52.0% w/w of the core,
- sodium alginate as antacid active ingredient in a percentage between 12.0% and 25.0% w/w of the core, and
- sodium bicarbonate as antacid active ingredient in a percentage between 4.0% and 9.0% w/w of the core.

The chewing gum tablet of the invention comprises sodium alginate in a percentage between 12.0% and 25.0% w/w of the core with the proviso that a $C_2$-$C_5$ polyol or poly($C_2$-$C_5$ alkylene glycol) is not included in the chewing gum tablet.

Preferable chewing gum tablet comprises sodium alginate in a percentage between 13.0% and 18.0%% w/w of the core with the proviso that a $C_2$-$C_5$ polyol or poly($C_2$-$C_5$ alkylene glycol) is not included in the chewing gum tablet.

In an embodiment, the calcium carbonate is in the core.

In the most preferred embodiment, the calcium carbonate is in the coating. The authors of the invention have found improved release, up to 100%, of calcium carbonate when it is in the coating.

The antacid chewing gum product of the invention is formulated in the form of a tablet having a core and a coating that fully or partially covers the core.

The present invention provides a non-swallowing antacid chewing gum tablet comprising three antacid components.

From an antacid point of view, the sum of antacid components as well as their distribution in the tablet provides a novel chewing gum with improved antacid action linked to improved organoleptic properties compared with Gaviscon Forte chewable tablets of the prior art.

The mechanism of the antacid effect of the tablets of the invention is known in the art, for example, from Gaviscon Forte chewable tablets. In the presence of gastric acid, alginates precipitate forming a gel, and the bicarbonate is converted to carbon dioxide which becomes entrapped within the gel precipitate, converting it into a foam which floats on the surface of the gastric contents. Besides, the bicarbonate and the carbonate neutralize the excess of acid in the stomach. It is well known in the art that sodium alginate is a good antacid component; however, its content is limited for the reasons stated above.

According to the chewing gum tablet of the invention, the sodium alginate is present in an amount of about 12% to about 25% w/w of the core. Despite the high amount of alginate used together with the presence of flavor components, disturbance in their release rate has not been detected. Moreover, the drawback of the "gag reflex" has been mitigated and/or removed.

Sodium alginate can be present in a weight ratio of gum base:sodium alginate of from about 2.3:1 to about 3.5:1, preferably of from about 2.6:1 to about 3:1.

It is understood by the skilled person in this field that the tablet, may also comprise further, optional components. The further, optional components may comprise one or more colorings, sweetenings, flavorings, emulsifiers, plasticizers, humectants, polishing agents, pH adjusting ingredients and fillers.

The invention also refers to a non-swallowed chewing gum tablet of the composition described above which is obtainable by an extruded method.

The chewing gum of the invention is preferably an extruded chewing gum tablet.

Preparation method

There are several known techniques in the art for preparing a chewing gum tablets.

The present inventors have found that the compression technique in this case can work on a laboratory scale. However, many problems arise when trying to scale on an industrial scale. For example, the sieving step causes greater friction between the particles and the temperature rises above 50 °C so the mass sticks to the sieve surfaces, and although the gum base in powder form is used to avoid sieving steps, compression does not run as fast as for conventional tablets. Compression techniques must be performed at a very low speed to control the rise in temperature. Also, the compression machine needs to be stopped several times to clean it.

It is theorized that the components of the chewing gum formulation of the invention to provide a non-swallowed chewing gum whose antacid components require to be released in a suitable profile during chewing action, limits the methods for preparing the chewing gum tablet of the invention. Main problems are on the difficulty for the product (the chewing gum core) to harden enough to be coated later. Likely, this may be the reason for not yet a chewing gum non-swallowed having a potent antacid effect is provided in the art.

It appears that the nature of the antacid components, as well as their distribution in the finished product, can adversely affect the production of the antacid chewing gums on an industrial scale.

One of the problems with the chewing gums is that the alginate, in a high quantity as is the case in the present invention, breaks down the gum base, and the unity of the gum base is hardly maintained after chewing.

Therefore, a further problem solved by the present invention is the physical stability of the non-ingestible chewing gum after releasing the antacid components so that it remains chewable.

The present inventors have found that the extrusion technique can be used for preparing the chewing gum tablet of the invention solving the physical stability problems as well as for preparing at industrial scale.

Thus, the chewing gum of the invention is preferably obtained by an extrusion method.

In a second aspect, the invention provides an extrusion process capable of easily preparing the non-ingestible chewing gum of the first aspect of the invention at industrial scale.

The extrusion process comprises the steps of:

a) preparing a mixture using the core ingredients in powder form;

b) extruding the mixture of step a) to obtain cores;

c) preparing a coating syrup using the coating ingredients;

d) coating the cores obtained in step b) with the coating syrup prepared in step c) to provide the non-ingestible antacid chewing gum.

Advantageously, the extrusion process solves the aforementioned temperature and adhesion problems and provides an antacid chewing gum tablet in which the gum base supports the release of the alginate without falling apart. The extrusion process yields an antacid chewing gum tablet consisting of two antacids in the core and one antacid in the coating.

In a third aspect, the invention is directed to the extruded chewing gum tablet obtained according to the extrusion process defined in the second aspect of the invention.

The extruded chewing gum tablet of the invention is fully acceptable to the consumer, with good organoleptic properties and strong antacid properties.

The present inventors have found that the extruded antacid chewing gum tablet is further physically stable after a time of chewing compared to the physical stability of the chewing gum tablet from compression methods in which the mass disintegrated and in which the consistency of the gum is lost during chewing.

In a fourth aspect, the extruded antacid chewing gum tablet of the invention is suitable for use as a non-ingestible antacid medication.

The extruded antacid chewing gum tablet of the invention is suitable for use in the prevention and/or treatment of a disorder or disease selected from acid regurgitation, heartburn, dyspepsia, gastric reflux, esophagitis and peptic ulceration.

In an embodiment, the extruded antacid chewing gum tablet of the invention is suitable for a group of patients having difficulty swallowing.

The foregoing and other features and advantages will become apparent from the following detailed description of the presently preferred embodiments, when read in conjunction with the accompanying examples.

**Brief Description of the Figures**

Figure 1. Raft strength (g) of a tablet of the invention and of Gaviscon Forte.
Figure 2. Resilience test of the invention and of Gaviscon Forte.
Figure 3. Average calcium dissolution (in mg) of the invention by time in minutes.

**Detailed Description of the Invention**

As used herein, the term "chewing gum" includes bubble gum and all types of chewing gum.

In the present invention, the terms "non-swallowed" and "non-ingestible" have been used indistinctly. Therefore, "non-swallowable" means "non-ingestible", or not to be ingested, not even after being chewed or kept in the mouth. The "chewing gum" of the invention comprises gum base which is meant to be disposed after being chewed, that is, the remaining solid, which is the gum base, is not-swallowed or not-ingested. The other ingredients of the chewing gum, in particular the active ingredients, are to be ingested, as they are released to the gastrointestinal tract during the chewing act.

The present invention is directed to an antacid chewing gum comprising a core and a coating that fully or partially covers the core. The antacid chewing gum is formulated in the form of a tablet.

All percentages used herein are weight percentages unless otherwise specified.

Core

The core comprises sodium alginate, sodium bicarbonate and gum base.

The weight of the sodium alginate may be about 12% to about 25% of the weight of the core. In a preferable embodiment, the weight of the sodium alginate may be about 13% to about 18% of the weight of the core.

The weight of the sodium bicarbonate may be about 4% to about 9% of the weight of the core. In a preferable embodiment, the weight of the sodium bicarbonate may be about 5.5% to about 7.5% of the weight of the core.

The weight of the gum base may be about 32% to about 52% of the weight of the core. In a preferable embodiment, the weight of the gum base may be about 38% to about 47% of the weight of the core.

The weight of one core can be from 1.2 to 1.9 g. The weight of one core may vary ± 5%. In a preferable embodiment, the weight of one core is from 1.5 to 1.7 g.

As stated above, in the most preferred embodiment, the core comprises the aforementioned components, and the calcium carbonate is in the coating. However, the present invention also contemplates the alternative in which the core further comprises the calcium carbonate.

Coating

In the most preferred embodiment, the calcium carbonate of the tablet is in the coating. In this preferred embodiment, the weight of the calcium carbonate may be about 20% to about 34% of the weight of the coating. In a preferable embodiment, the weight of the calcium carbonate may be about 22% to about 32% of the weight of the coating. In a still preferable embodiment, from about 24% to about 30% of the weight of the coating.

In a preferred embodiment, the coating fully coats the core. In another embodiment the coating partially coats the core.

Chewing gum tablet

The weight of the core may be about 60% to about 80% of the weight of the chewing gum tablet. In a preferable embodiment, the weight of the core may be about 65% to about 75% of the weight of the chewing gum tablet.

The weight of the sodium alginate may be about 8% to about 14% of the weight of the chewing gum tablet. In a preferable embodiment, the weight of the sodium alginate may be about 9% to about 12.5% of the weight of the chewing gum tablet.

The weight of the sodium bicarbonate may be about 2% to about 6% of the weight of the chewing gum tablet. In a preferable embodiment, the weight of the sodium bicarbonate may be about 3.5% to about 5.2% of the weight of the chewing gum tablet.

The weight of the gum base may be about 25% to about 35% of the weight of the chewing gum tablet. In a preferable embodiment, the weight of the gum base may be about 27% to about 32% of the weight of the chewing gum tablet.

The weight of the coating may be about 20% to about 40% of the weight of the chewing gum tablet. In a preferable embodiment, the weight of the coating may be about 25% to about 35% of the weight of the chewing gum tablet.

The weight of the calcium carbonate may be about 6% to about 10% of the weight of the chewing gum tablet. In a preferable embodiment, the weight of the calcium carbonate may be about 7% to about 9% of the weight of the chewing gum tablet. In a still more preferable embodiment, the weight of the calcium carbonate may be about 7.5% to about 8.5% of the weight of the chewing gum tablet.

The weight of one single chewing gum tablet is from 2.1 to 2.5 g. The weight of one single tablet may vary ± 6%. In a preferable embodiment, the weight of one single chewing gum tablet is from 2.2 to 2.4 g.

The one single tablet weights and includes sodium alginate from 237 to 263 mg, sodium bicarbonate from 100 to 113 mg, and calcium carbonate from 180 to 207 mg.

Various grades of sodium alginate are commercially available that yield aqueous solutions of varying viscosity. Preferable sodium alginate is of low viscosity grade, particularly from 200 to 800 mPa.s. Generally, the viscosity of a 10% weight/volume aqueous solution, when determined on a Brookfield RVT viscometer using spindle number 3 at 20 r.p.m. at 20 °C, falls within the range of 200 to 800 mPa.s. Preferable sodium alginate is selected having a viscosity between 300 and 700 mPa.s. See more detailed information in "International Journal of Pharmaceutics 294 (2005) 137-147'.

The insoluble gum base generally comprises elastomers, resins, fats and oils, waxes, softeners and inorganic fillers. The elastomers may include polyisobutylene, isobutyiene-isoprene copolymer, styrene butadiene rubber and natural latexes such as chicle. The resins may include polyvinylacetate and terpene resins. Low molecular weight polyvinylacetate is a preferred resin. Fats and oils may include tallow, soybean and cottonseed oils, hydrogenated vegetable oils and cocoa butter. Gum bases typically also contain antioxidants, colors and emulsifiers. The present invention contemplates employing any commercially acceptable gum base.

It is understood by the skilled person in this field that the core and the coating may also comprise further, optional components.

Preferably, the core further comprises at least one of sweeteners, humectants, bulking agents and flavoring agents.

Preferably, the coating further comprises at least one of coloring agents, sweeteners, flavoring agents, emulsifiers and polishing agents.

The preferable sweeteners are selected from the group consisting of isomalt, isomaltidex, maltitol, acesulfame potassium, aspartame, saccharin, Lycasin (80/55 maltitol syrup) and mixtures thereof.

In a preferred embodiment, humectants may be selected from glycerin, Lycasin (80/55 maltitol syrup) and mixtures thereof.

In a preferred embodiment emulsifier is selected from Arabic gum.

Manufacture of the chewing gum tablet

The extrusion process for manufacturing the non-swallowed, antacid chewing gum tablet of the first aspect of the invention comprises the steps of:

a) preparing a mixture with the core ingredients;
b) extruding the mixture obtained in step a) and forming the cores;
c) preparing a coating syrup with the coating ingredients;
d) coating the extruded and formed cores of step b) using the coating syrup of step c) to obtain the non-swallowed, antacid chewing gum tablet.

- Step a - Preparing a mixture with the core ingredients.

The core ingredients are mixed with the gum base in a mixer. Due to the mixing process the temperature increases and favors that all the ingredients are mixed and/or melted together. Preferably, the temperature reached during the process is around 30°C - 55°C.

For example, the mixing process can be done in a double sigma mixer.

- Step b - Extruding the mixture obtained in step a) and forming the cores.

The chewing gum mixture obtained in step a) is cut into portions to feed an extruder that will produce a thick sheet of chewing gum that a forming machine will laminate and produce precut cores at the proper thickness that once conditioned will be coated.

Preferably, the temperature during this step is below 50°C.

For example, this step is performed in rolling and scoring machines.

Preferably, the formed cores stand until getting harden before being coated in step d. Preferably they stand at controlled conditions of humidity and temperature. Preferably they stand at controlled conditions of less than 60% of relative humidity (RH). Preferably they stand at controlled conditions of temperature of less than 25°C, more preferably between 14 and 22°C.

- Step c - Preparing a coating syrup with the coating ingredients.

A coating syrup is preferably an antacid coating syrup which contains calcium carbonate.

Preferably, the calcium carbonate is present in powder form in the antacid syrup. Preferable calcium carbonate has a purity equal or higher than 99%.

In one embodiment, the antacid coating syrup comprises calcium carbonate, a sweetener agent, water, and a syrup mucilage.

The syrup mucilage is a thick aqueous solution that can be prepared with for example a gum such as gum Arabic or with a dextrin. The syrup mucilage, in the antacid coating syrup, is used to suspend insoluble substances and to increase viscosity. The syrup mucilage preferably comprises water and a coating agent. In a preferred embodiment the coating agent is an emulsifying agent or a plasticizer agent. In a preferred embodiment the coating agent is Arabic gum or a dextrin. Preferably, the mucilage comprises water and a coating agent in a ratio of about 55:45 and 45:55 % w/w, more preferably in a ratio of about 50:50 % w/w. In one embodiment, the syrup mucilage is water and Arabic Gum in a ratio of 50:50 % w/w.

The sweetener agent is preferably isomalt.

In an alternative embodiment it also contains flavour.

In a preferred embodiment, the coating comprises various coatings or sub-coatings. In a preferable embodiment, three coating or sub-coatings syrups are prepared and used in subsequent coatings for obtaining a first sealing coating, a second antacid coating and a third finishing coating, for polishing and shining appearance. In another embodiment, where calcium carbonate is only present in the core and not in the coating, there is not a second antacid coating.

In a preferred embodiment, the respective coating syrups for obtaining each of the coatings (or sub-coatings) included in the coating are prepared as follows:

8

In one embodiment, the first coating syrup is the sealing coating syrup which is to be applied to cover the hardened cores formed in step b).

The sealing coating syrup is obtained from a mixture comprising water and a coating agent. In a preferred embodiment the coating agent is an emulsifying agent or a plasticizer agent. In a preferred embodiment the coating agent is Arabic gum or a dextrin. In a preferable embodiment this initial syrup preferably comprises water and a coating agent in a ratio from 75:25 to 65:35 %w/w. Preferably, it comprises water and a coating agent in a ratio of about 70:30 %w/w. In a preferred embodiment the coating agent is Arabic gum or a dextrin. In a more preferred embodiment, this initial syrup is a mucilage comprising water and Arabic Gum in a ratio of about 70:30 % w/w.

In one embodiment, the sub-coating having the antacid properties is the second coating. The antacid sub-coating is obtained from applying the antacid coating syrup comprising calcium carbonate.

In one embodiment, a third coating syrup is prepared for a third sub-coating, said coating being a finishing coating for polishing and shining appearance. The finishing coating syrup preferably comprises water, a coloring agent and a syrup mucilage. In a preferred embodiment it also comprises flavouring agents.

The prepared syrup mucilage for this finishing coating preferably comprises water and a coating agent. In a preferred embodiment the coating agent of the finishing coating is an emulsifying agent or a plasticizer agent. In a preferred embodiment the coating agent is Arabic gum or a dextrin. Preferably, the syrup mucilage comprises water and a coating agent in a ratio of about 55:45 and 45:55 %w/w, more preferably in a ratio of about 50:50 %w/w. Preferably, the syrup mucilage comprises water and Arabic Gum in a ratio of about 50:50% w/w.

In one embodiment, all the water content of the syrup coatings is dried during coating process. In other embodiment, less than 1.1% of residual water remains in the final chewing gum tablets.

- Step d - Coating the cores of step b) using the coating syrup of step c) to obtain the non-swallowed, antacid chewing gum tablet.

Once the chewing gum in the cores is hardened, that is, the cores formed in step b) are hardened, they are ready to be coated. This coating operation preferably comprises repeating the following process: i) wetting the cores with the coating syrup prepared in step c); ii) distributing the syrup; and iii) drying.

In a preferred embodiment the coating operation comprises applying various coatings or sub-coating syrups.

In a preferred embodiment the coating operation comprises a first step of applying the sealing coating syrup as prepared in step c) for coating the cores. This coating operation comprises repeating the following process: i) wetting the cores with the sealing coating syrup prepared in step c; ii) distributing the syrup; and iii) drying.

In a preferred embodiment where the tablet comprises calcium carbonate in the coating, the coating operation comprises a step of applying the antacid coating syrup as prepared in step c) covering the dried sealing coating. This embodiment of coating operation comprises repeating the following process: i) wetting the dried cores coated with the sealing coating with the antacid coating syrup prepared in step c; ii) distributing the syrup; and iii) drying.

In a preferred embodiment the coating operation comprises a further step of applying the finishing coating syrup as prepared in step c), covering the antacid coating. This coating operation comprises repeating the following process: i) wetting the dried cores coated with the antacid coating with the finishing coating syrup prepared in step c; ii) distributing the syrup; and iii) drying. In an alternative embodiment where the tablet comprises calcium carbonate only in the core, the coating operation comprises a further step of applying the finishing coating syrup as prepared in step c), covering the sealing coating.

In a preferred embodiment, the coating process i) of wetting the cores (uncoated or already coated) is performed by adding the coating syrup to the cores loaded in a coating pan.

In a preferred embodiment, the coating process ii) of distributing the syrup is performed by leaving the coating pan rotate to distribute the syrup.

In a preferred embodiment, the coating process iii) of drying is performed by the following preferred process: by adding Isomalt in powder and/or by blowing air, the air preferably at a temperature of about 10 to 30 °C and relative humidity of about 10% to 30%.

Subsequent manufacturing steps may comprise passing the coated chewing gums through a metal detector, wrapping, packaging, fill bottles, etc.

## Examples

Hereinafter, the present invention is described in more detail and specifically with reference to the Examples and Figures, which however are not intended to limit the present invention. The chewing gum tablets of the invention are dismissed after being chewed thoroughly, that is, the chewing gum is not swallowed.

Examples

**Example 1 - Core preparation -**

In this embodiment, the core weights 1,600 mg. As the core weight may vary $\pm$ 5%, this embodiment also encompasses this variation by decreasing or increasing this range in each ingredient.

The core ingredient quantities from table 1 below are added to a doble sigma mixer.

**Table 1**

| Core Ingredients | % (wt) | Amount (mg) |
|---|---|---|
| Saccharin | 0.200 | 3.2 |
| Glycerin | 0.500 | 8.0 |
| Isomalt | 24.769 | 396.3 |
| Lycasin 80/55 Maltitol syrup | 3.500 | 56.0 |
| Acesulfame | 0.100 | 1.6 |
| Granular Aspartame | 0.100 | 1.6 |
| Gum Base ZEUS-T | 42.000 | 672.0 |
| Sodium Bicarbonate | 6.656 | 106.5 |
| Sodium Alginate | 15.625 | 250.0 |
| Eucamentol liquid | 1.550 | 24.8 |
| Luctadry Eucamentol powder | 5.000 | 80.0 |
| Total | 100.000 | 1,600 |

When the mixing process is finalized, the mass is unloaded. The above mass is cut in portions to feed the extruder that produces a thick sheet. The rollers of the subsequent forming machine will provide size and format the cores. The cores are kept in a conditioning room at controlled conditions of humidity (<60%RH) and temperature (14°C-22°C) to harden.

**Example 2 - Coating syrups -**

Initial syrup: Example of initial coating mucilage is Gum Arabic:Water, 30:70.

Antacid syrup: An example of antacid syrup comprises:

| Carbonate syrup | % (wt) |
|---|---|
| Water | 27.500 |
| Isomalt | 34.117 |
| Gum Arabic | 1.500 |
| Calcium Carbonate | 36.883 |
| Total | 100.00 |

An example of a finishing syrup comprises:

| Finishing Syrup | % (wt) |
|---|---|
| Water | 30.550 |
| Isomalt | 67.900 |
| Gum Arabic | 1.500 |
| Total | 100.00 |

**Example 3 - Coating the cores -**

The term "antacid syrup" is understood the sub-coating syrup that includes the calcium carbonate as antacid active ingredient of the coating.

The term "pellet" means a coated core, in which the core is coated by the total amount of the coating or only by a part of the total amount used in the coating.

1. The coating pan or coater was loaded with the cores of Example 1 when they were hard enough, usually after 2 to 14 days on the conditioning room (T< 25°C; RH < 60%). The unit weight is 1.6 g per unit (± 5%).
2. The coating operation has 3 phases. The coater is always rotating.

a. The initial syrup (mucilage of Water:Arabic Gum 70:30) of Example 2 was applied over the dried cores by applying layers as follows:

i. Adding the syrup to wet the cores
ii. Leaving the pan rotate to distribute the syrup
iii. Drying adding Isomalt in powder

Therefore obtaining the cores coated with Arabic Gum and Isomalt.
b. The antacid syrup (calcium carbonate syrup) was applied over the dried cores coated with Arabic Gum and Isomalt as follows:

i. Adding the syrup to wet the cores
ii. Letting the coating pan rotate to distribute the syrup
iii. Dry the syrup:

1. With addition of Isomalt powder in the first 3 syrup applications or
2. Blowing air at 10 °C-30°C, RH 10% - 30% for the following syrup layers.

These steps (i, ii and iii) were repeated until the desired weight of calcium carbonate was reached and:
iv. when the pellets (coated cores) reached around 1.89 g, acesulfame was dissolved in the syrup for one layer application, added to the coating pan to follow steps i, ii and iii.
v. When the pellets (coated cores) reached around 1.92 g, the flavor was added in two applications, dried by isomalt powder, separated by two normal applications.
vi. The coating was continued until the weight of the pellet is around 2.15 g.

c. Finishing

The process was continued with same steps i, ii and iii until the weight is 2.3 g per pellet but using the finishing syrup of Example 2.
3. Polishing
To the obtained pellets, half of the carnauba wax was added. The coating pan continued rotating for a while. The remaining carnauba wax was finally added to the pellets in the rotating coating pan, obtaining final shining pellets (the antacid chewing gum tablet).

The unit weight of the final tablet or pellet of the Example is 2.3 g (± 6%).

**Example 4 - Coating ingredients -**

The table below includes an Example of a final composition of the coating in the manufactured antacid chewing gum of the invention. The coating weights 700 mg although it may vary.

**Table 2**

| Coating ingredients | % (wt) | Amount (mg) |
|---|---|---|
| Calcium Carbonate | 26.983 | 188.881 |
| Isomalt | 12.396 | 86.772 |
| Arabic Gum | 2.212 | 15.484 |
| Carnauba Wax | 0.329 | 2.303 |
| Acesulfame | 0.036 | 0.252 |
| Isomaltidex | 56.073 | 392.511 |
| Eucamentol liquid | 1.971 | 13.797 |

(continued)

| Coating ingredients | % (wt) | Amount (mg) |
|---|---|---|
| Total | 100.00 | 700.00 |

The content of Table 1 that includes the core ingredient quantities and the content of Table 2 that includes the coating ingredient quantities is summarized below together with the weight partial percentage of the core and of the coating as well as the global percentage of the total chewing gum tablet.

| Material | Unitary Amount (mg) | Partial Formula (% p/p) | Global Formula (% p/p) |
|---|---|---|---|
| **CORE** | | | |
| **Sodium Alginate (API)** | **250** | **15.63** | **10.87** |
| **Sodium Bicarbonate** | **106.5** | **6.66** | **4.63** |
| Base Gum | 672 | 42 | 29.22 |
| Isomalt | 396.3 | 24.77 | 17.23 |
| Lycasin 80/55 Maltitol syrup | 56 | 3.5 | 2.43 |
| Glycerin | 8 | 0.5 | 0.35 |
| Saccharin | 3.2 | 0.2 | 0.14 |
| Luctadry Eucamentol powder | 80 | 5 | 3.48 |
| Eucamentol liquid | 24.8 | 1.55 | 1.08 |
| Granular Aspartame | 1.6 | 0.1 | 0.07 |
| Acesulfame | 1.6 | 0.1 | 0.07 |
| **TOTAL CORE** | **1,600** | **100** | **69.6** |
| **COATING** | | | |
| **Calcium Carbonate (API)** | **187.5** | 26.79 | **8.15** |
| Arabig Gum | 15.5 | 2.21 | 0.67 |
| Isomalt | 86.8 | 12.4 | 3.77 |
| Canauba Wax | 2.3 | 0.33 | 0.1 |
| Acesulfame | 0.2 | 0.03 | 0.01 |
| Isomaltidex | 393.9 | 56.27 | 17.13 |
| Eucamentol liquid | 13.8 | 1.97 | 0.6 |
| **TOTAL COATING** | **700** | **100** | **30.4** |
| **TOTAL CHEWING GUM TABLET** | **2,300** | **-** | **100** |

**Example 5- Comparative studies with other antacid products -**

The following methods are published on *International Journal of Pharmaceutics*, 2005, 294, 137-147, as well as some of the results of the products in the market. Gaviscon Forte's results were obtained in the laboratory by the inventors as the examples of the invention.

The speed and character of raft formation of the antacid chewing gum of Example 3 is compared in Table 3.

**Table 3 - Speed and character of raft formation by product -**

| Product | Formation speed | Flotation | Coherence |
|---|---|---|---|
| Algicon | Immediate | Partial | Poor |
| Gastrocote | Slow | Partial | Good |

(continued)

| Product | Formation speed | Flotation | Coherence |
|---|---|---|---|
| Gaviscon Advance | Immediate | Complete | Good |
| Gaviscon Liquid | Immediate | Complete | Good |
| Gaviscon Regular Strength (USA) | Slow | Very low | Poor |
| Gaviscon Extra Strength (USA) | Slow | Very low | Poor |
| Mylanta Heartburn Relief | Immediate | Complete | Good |
| Peptac | Slow | Partial | Good |
| Rennie Duo | Immediate | Complete | Good |
| Gaviscon Forte | Immediate | Complete | Good |
| Example 3 (invention) | Inmediate | Complete | Good |

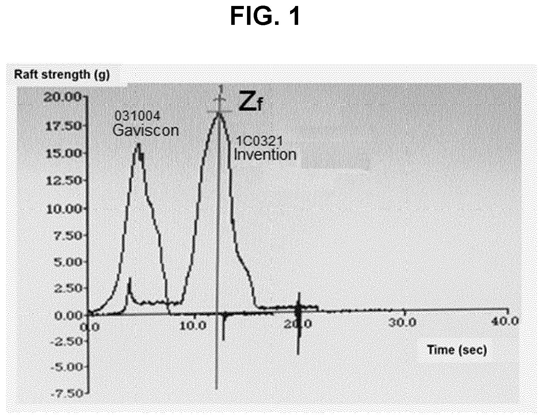

Table 4 below shows the raft strength (g), and raft volume (mL) of the antacid chewing gum of Example 3 as compared with other antacid products in the market. Results obtained for Gaviscon Forte and Example 3 of the invention are shown in Figure 1.

**Table 4 - Raft strength (g), and raft volume (mL) -**

| Product | Raft strength(g) | Raft volume (ml) |
|---|---|---|
| Algicon | 1.6 | 11.2 |
| Gastrocote | 7.9 | 21.1 |
| Gaviscon Advance | 16.5 | 25.8 |
| Gaviscon Liquid | 12.9 | 88.7 |
| Gaviscon Regular Strength (USA) | 1.8 | 5.8 |
| Gaviscon Extra Strength (USA) | 1.1 | 10.4 |
| Mylanta Heartburn Relief | 4.6 | 36.8 |
| Peptac | 10.8 | 42.3 |
| Rennie Duo | 4.1 | 28.0 |
| Gaviscon Forte | 15.9 | 47.0 |
| Example 3 (invention) | 18.5 | 52.8 |

**Example 6- Resilience** -

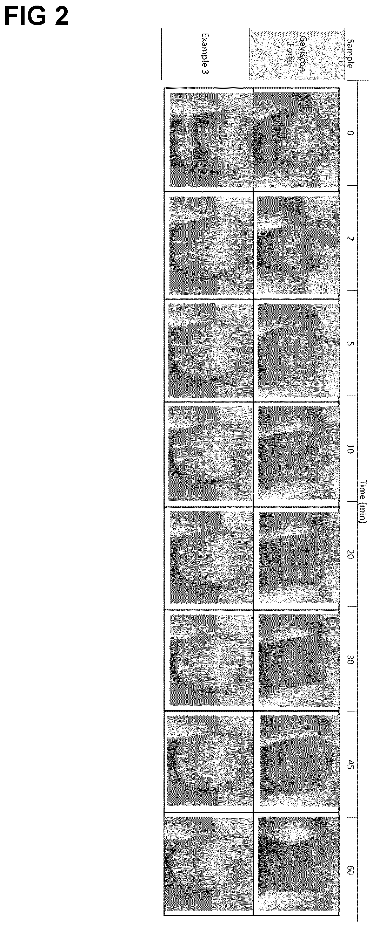

Raft strength is also related to raft resilience. The resistance to break-up under the conditions of movement in the raft resilience test is improved with increase in raft strength.
In Example 5, Table 4, Gaviscon Forte and the chewing gum of Example 3 show high raft strength.
As shown in Figure 2, Gaviscon Forte and the chewing gum of Example 3 present high and similar resistance to raft break-up in the resilience test.

**Example 7- Calcium dissolution** -

The release of Calcium carbonate for the chewing gum of the invention is determined by measure of Calcium by ICP-OES method according to the European Pharmacopoeia (method 2.9.3): Dissolution test for solid oral dosage forms, under the conditions described in section 2.1. The test was performed for six samples (V1-V6). See below Table 5.

**Table 5**

| Time | V1 | V2 | V3 | V4 | V5 | V6 | Average | SD | RSD | Max | Min |
|---|---|---|---|---|---|---|---|---|---|---|---|
| 0 min | 0.00 | 0.00 | 0.00 | 0.00 | 0.00 | 0.00 | 0.00 | 0.00 | 0.0% | 0.00 | 0.00 |
| 5 min | 224.37 | 216.96 | 149.19 | 199.76 | | 233.08 | 204.67 | 33.35 | 16.3% | 233.08 | 149.19 |
| 10 min | 219.94 | 197.69 | 198.64 | 221.74 | 203.08 | 211.26 | 208.72 | 10.55 | 5.1% | 221.74 | 197.69 |
| 20 min | 247.45 | 192.92 | 179.80 | 172.87 | 197.87 | | 198.18 | 29.30 | 14.8% | 247.45 | 172.87 |

(continued)

| Time | V1 | V2 | V3 | V4 | V5 | V6 | Average | SD | RSD | Max | Min |
|---|---|---|---|---|---|---|---|---|---|---|---|
| **30 min** | 205.02 | 173.32 | 202.23 | 185.57 | 226.34 | 179.34 | 195.30 | 19.69 | 10.1% | 226.34 | 173.32 |

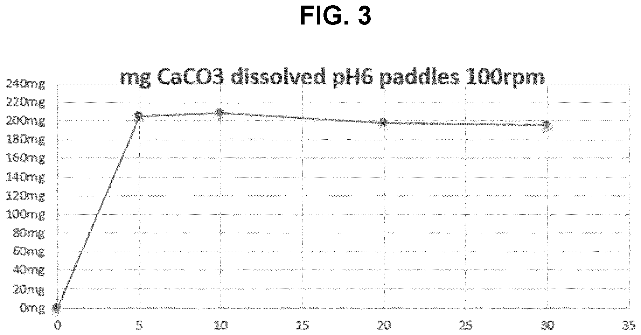

Figure 3 shows the results of the average column.

**Example 8- Acid Neutralizing Capacity -**

The acid-neutralizing capacity (ANC) of an antacid is the amount of acid that can be neutralized and is determined by a back titration method to a set endpoint of pH 3.5.
Solutions preparations

- Purified water

- 0.5N Sodium hydroxide: Around 20 g sodium hydroxide is weighed accurately and transferred into a clean 1000 mL volumetric flask. It is then dissolved and diluted up to the mark with carbon dioxide free water.

- 1.0N Hydrochloric acid: take 87ml of hydrochloric acid 37% and transfer into a 1000ml volumetric flask. It is then dissolved and diluted up to the mark with carbon dioxide free water.

Sample preparation

Chewing gum:

- Weigh one chewing gum and grinded into a fine powder or cut into the minimum pieces possible.
- Transfer into a suitable beaker (150-250ml) and add 70ml of purified water.
- Stir and homogenize the solution for 1 minute.
- Add accurately 30ml of 1.0N hydrochloric acid and stir well during 15min exactly.
- After that the excess hydrochloric acid is titrated immediately with NaOH 0.5N until a stable pH of 3.5 is achieved (during about 10-15 seconds).

Calculations

$$Total\ mEq = (30\ x\ N_{HCl}) - (V_{NaOH}\ x\ N_{NaOH})$$

where:

$N_{HCl}$ = Hydrochloric acid normality
$V_{NaOH}$ = Volumen of NaOH used
$N_{NaOH}$ = Sodium hydroxide normality

Results of two tablets are shown below:

| Weigh (mg) | Samples | N HCl | V NaOH | N NaOH | total mEq |
|---|---|---|---|---|---|
| 2,282.1 | Chewing gum tablet invention 1C-03/21_M1 | 1 | 51.9 | 0.5 | 4.1 |
| 2,219.6 | Chewing gum tablet invention 1C-03/21_M2 | 1 | 50.3 | 0.5 | 4.9 |
| Average | | | | | 4.5 |

Results of the acid-neutralizing capacity (ANC) of the chewing gum tablet of the invention are similar to the values of the prior art, but moreover the invention chewing gum tablet is formulated to be chewed without the need to be swallowed in order to reveal with high efficiency the antacid effect.

**Example 9 - Chewing gum tablet containing all the APIs in the core** -

An antacid chewing gum containing all the APIs in the core was manufactured by compression containing the following ingredients:

**Table 6**

| Name of ingredient | Quantity / tablet (mg) |
|---|---|
| *Active ingredient* | |
| Sodium alginate | 250.00 mg |
| Calcium carbonate | 197.38 |
| Sodium hydrogencarbonate | 106.50 |
| *Excipients* | |
| Gumbase | 915.62 |
| Isomalt | 50.08 |
| Acesulfame K | 4.50 |
| Saccharin | 2.00 |
| Silicium dioxide | 12.00 |
| Magnesium stearate | 16.00 |
| Peppermint Durarome Flavor | 85.00 |
| Peppermint oil Naefco | 16.50 |
| *Core weight* | *1655.68* |
| Opadry | 49.67 |
| *Tablet sum* | *1705.35* |

The following raw material were charged into the High Shear Mixer (HSM): Sodium alginate, calcium carbonate, sodium hydrogen carbonate, isomalt, acesulfame, saccharin, sillicum dioxide and flavors. The ingredients were mixed for 15 minutes at impeller speed of 300 rpm and chopper off.
The gum base was sieved in Quadro Comil U20 trough a sieve of 1.80 mm (in another example a 1.905mm sieve was used).
The sieved gum was charged into the HSM and mixed with the rest of ingredients for 15 minutes at impeller speed of 600 rpm and chopper speed 1500 rpm. More flavor was sprayed and mixed.
The mixture was sieved and blended with magnesium stearate.
The mixture was charged in a tableting machine and tablets obtained by compression.
The tablets were coated in a coating pan with a previously prepared homogeneous solution of Opadry/water 10:90.
The above antacid gum was manufactured at lab scale and provided good results. However, the scale-up of the manufacturing process in large scale was inviable.
The inventors consider that the temperatures reached by the compression process in large amounts made the above mixture hard to manipulate at large scale.

**Claims**

1.    A non-swallowed, antacid chewing gum tablet comprising a core and a coating, **characterized in that** the tablet comprises calcium carbonate as antacid active ingredient, and, the core comprises:

- gum base in a percentage between 32.0% and 52.0% w/w of the core,
- sodium alginate as antacid active ingredient in a percentage between 12.0% and 25.0% w/w of the core, and
- sodium bicarbonate as antacid active ingredient in a percentage between 4.0% and 9.0% w/w of the core.

2. The chewing gum tablet according to claim 1, wherein the calcium carbonate of the tablet is in the coating.

3. The chewing gum tablet according to any one of preceding claims, wherein the gum base:sodium alginate % w/w ratio is between 2.3:1 and 3.5:1.

4. The chewing gum tablet according to any one of preceding claims, wherein the calcium carbonate is in a percentage between 22.0% and 32.0%% w/w of the coating.

5. The chewing gum tablet according to any one of the preceding claims, wherein one single chewing gum tablet comprises calcium carbonate between 6.0 and 10.0 % w/w of the tablet.

6. The chewing gum tablet according to any one of the preceding claims, wherein one single tablet weights from 2,100 to 2,500 mg and includes sodium alginate from 237 to 263 mg, sodium bicarbonate from 100 to 113 mg, and calcium carbonate from 187 to 207 mg.

7. The chewing gum tablet according to any one of the preceding claims, wherein one single core weights from 1,200 to 1,900 mg.

8. The chewing gum tablet according to any one of the preceding claims, wherein the coating fully or partially coats the core.

9. A chewing gum tablet according to any one of previous claims obtainable by an extrusion process.

10. An extrusion process for manufacturing a non-swallowed, antacid chewing gum tablet as defined in claims 1 to 9, wherein the process comprises the steps of:

a) preparing a mixture with the core ingredients;
b) extruding the mixture obtained in step a) and forming the cores;
c) preparing a coating syrup with the coating ingredients;
d) coating the extruded and formed cores of step b) using the coating syrup of step c) to obtain the non-swallowed, antacid chewing gum tablet.

11. The process according to claim 10, wherein extruded and formed core of step b) is standing until getting harden prior to coating of step d).

12. The process according to any one of claims 10-11, wherein the calcium carbonate is in the coating and the process comprises the steps of:

a) preparing a mixture with the core ingredients including the gum base, the sodium alginate and the sodium bicarbonate,
b) extruding the mixture obtained in step a) and forming the cores;
c) preparing a coating syrup with the coating ingredients including the calcium carbonate;
d) coating the extruded and formed cores of step b) using the coating syrup of step c) to obtain the non-swallowed, antacid chewing gum tablet.

13. The process according to any one of claims 10 to 12, wherein the preparation of the coating syrup of step c) includes an initial syrup and/or an antiacid syrup and a finishing syrup.

14. The chewing gum tablet composition as defined in claims 1 to 9 for use as a non-ingestible antacid medication.

15. The chewing gum tablet composition according to claim 14 for use in a method for the prevention and/or the treatment of a disorder or disease selected from acid regurgitation, heartburn, dyspepsia, gastric reflux, oesophagitis and peptic

ulceration.

# FIG. 1

# FIG 2

# FIG. 3

mg CaCO3 dissolved pH6 paddles 100rpm

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0217966 A
- WO 0217731 A
- WO 02051259 A
- WO 0195738 A
- WO 03068246 A
- WO 2000030466 A

**Non-patent literature cited in the description**

- *International Journal of Pharmaceutics*, 2005, vol. 294, 137-147
- *nternational Journal of Pharmaceutics*, 2005, vol. 294, 137-147